# EUROPEAN PATENT APPLICATION

(11) **EP 4 835 058 A1**
(43) Date of publication of application: **04.11.2026**
(21) Application number: 24911401.8
(22) Date of filing: 27.12.2024
(51) Int. Cl.: C12N 5/10, A61K 40/42, A61K 35/17, A61K 39/00, A61P 35/00

(54) **MODIFIED INKT CELL**

(30) Priority: 29.12.2023 CN 202311870737
(71) Applicant: SPH Biotherapeutics (HK) Limited, New Territories Hong Kong 999077 (HK); SPH Biotherapeutics (Shanghai) Limited, Shanghai 200072 (CN)
(72) Inventor: ZHANG, Hua, Hong Kong 999077 (CN); HUA, Jian, Shanghai 200072 (CN); XU, Feng, Hong Kong 999077 (CN); CUI, Yuzhu, Hong Kong 999077 (CN); QUAN, Dexian, Shanghai 200072 (CN); DENG, Linyan, Hong Kong 999077 (CN); WAN, Yuhua, Shanghai 200072 (CN)
(74) Representative: Elzaburu S.L.P.
(86) International application number: PCT/CN2024/142983
(87) International publication number: WO 2025/140480

(57) **Abstract**

A modified iNKT cell, which , compared with an unmodified iNKT cell, lacks or inhibits the expression of a negative immunoregulatory gene, wherein the gene is selected from one or more of the froup consisting of PD-1, Tim-3, CTLA-4, CD74 and TGF- β R, and the lack or inhibition of the expression of the negative immunoregulatory gene is realized by means of the technique of intracellular retention/degradation of a membrane protein. Further provided is the use of the iNKT cell and a composition thereof.

## Description

### Technical Field

The present application relates to the field of biomedicine and pharmaceuticals, specifically to a modified iNKT cell and its use.

### Background Art

Invariant natural killer T cells (iNKT cells) are innate-like CD1d-restricted T cells that express an invariant T cell receptor (iTCR). They undergo a unique differentiation program in the thymus and acquire NK-like properties, including potent cytotoxicity, rapid cytokine secretion, and efficient trafficking into tissues. iNKT cells are among the earliest cell types to respond to pathogenic stimuli, and their cytokine production helps determine the course of the immune response. Due to their anti-tumor efficacy, iNKT cells have recently emerged as potential targets for immunotherapy.

Membrane protein intracellular retention technology is a non-gene-editing technique based on the principle that retention sequences have corresponding receptors on the Golgi membrane. Once such a sequence enters the Golgi apparatus, it binds to the KDEL receptor on the Golgi and is packaged into retrograde vesicles that transport it back to the endoplasmic reticulum. Thus, an antibody fused with a KDEL retention sequence can be effectively retained within the endoplasmic reticulum, thereby downregulating antibody complexes on the cell surface and achieving a loss-of-function effect similar to gene editing, thus avoiding GvHD.

Currently, there is limited research on developing universal CAR-iNKT cells using the non-gene-editing membrane protein intracellular retention technology. There is an urgent need to combine these two technologies to develop relevant products for disease treatment.

### Summary of the Invention

The present application provides a modified iNKT cell that, compared to an unmodified iNKT cell, lacks or inhibits expression of one or more negative immune regulator genes selected from the group consisting of PD-1, Tim-3, CTLA-4, CD74, and TGF-βR, wherein said lack or inhibition of expression of the negative immune regulator genes are achieved by membrane protein intracellular retention technology. Compared to unmodified iNKT cells, the modified iNKT cells provided herein exhibit significantly inhibited expression of negative immune regulator genes; demonstrate significant inhibitory and cytotoxic functions upon secondary stimulation with tumor cells; show markedly increased survival rates in vitro; and further enhanced tumor-killing capacity when co-expressing cytokines. The modified iNKT cells provided herein not only stably inhibit the expression of negative immune regulator genes but also possess potent tumor-killing activity and exhibit significantly improved survival rates, yielding unexpectedly advantageous technical effects.

In one aspect, the present application provides a modified iNKT cell that, compared to an unmodified iNKT cell, lacks or inhibits expression of one or more negative immune regulator genes selected from the group consisting of PD-1, Tim-3, CTLA-4, CD74, and TGF-βR, wherein said lack or inhibition of expression of the negative immune regulator genes are achieved by membrane protein intracellular retention technology.

In certain embodiments, the membrane protein intracellular retention technology involves directly or indirectly linking a targeting sequence to the N-terminus or C-terminus of the negative immune regulator gene. In certain embodiments, the membrane protein intracellular retention technology involves directly or indirectly linking a targeting sequence to the C-terminus of the negative immune regulator gene. In certain embodiments, the membrane protein intracellular retention technology involves directly or indirectly linking a targeting sequence to the N-terminus of the negative immune regulator gene. In certain embodiments, the indirect linkage uses (GGGGS)₄ as a linker.

In certain embodiments, the negative immune regulator gene is PD-1. In certain embodiments, the amino acid sequence of said PD-1 is as set forth in SEQ ID NO: 12 or SEQ ID NO: 19.

In certain embodiments, the targeting sequence is selected from the group consisting of an endoplasmic reticulum retention sequence, a Golgi retention sequence, an E3 ubiquitin ligase binding sequence, a proteasome targeting sequence, and a lysosomal targeting sequence.

In certain embodiments, the targeting sequence is an endoplasmic reticulum retention sequence. In certain embodiments, the targeting sequence is a lysosomal targeting sequence.

In certain embodiments, the amino acid sequence of the endoplasmic reticulum retention sequence comprises one or more amino acid sequences selected from the group consisting of KDEL (SEQ ID NO: 9), KKMP (SEQ ID NO: 10), and AEKDEL (SEQ ID NO: 11).

In certain embodiments, the amino acid sequence of the lysosomal targeting sequence is as set forth in SEQ ID NO: 8.

In certain embodiments, the iNKT cell expresses a CAR.

In certain embodiments, the CAR comprises a targeting moiety that specifically binds to an antigen.

In certain embodiments, the antigen is selected from one or more antigens from the group consisting of GPC3, CLDN18.2, ROR1, and AXL.

In certain embodiments, the antigen is GPC3. In certain embodiments, the antigen is ROR1.

In certain embodiments, the targeting moiety is an scFv. In certain embodiments, the targeting moiety is a VHH.

In certain embodiments, the CAR comprises a signal peptide.

In certain embodiments, the signal peptide is derived from the CD8 protein.

In certain embodiments, the signal peptide is derived from IL-2.

In certain embodiments, the CAR comprises a hinge region, wherein the hinge region comprises a hinge region derived from one or more proteins selected from the group consisting of CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

In certain embodiments, the hinge region is derived from CD28. In certain embodiments, the hinge region is derived from CD8.

In certain embodiments, the CAR comprises a transmembrane domain, wherein the transmembrane domain comprises a transmembrane domain derived from one or more proteins selected from the group consisting of CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

In certain embodiments, the transmembrane domain is derived from CD28.

In certain embodiments, the CAR comprises a co-stimulatory domain, wherein the co-stimulatory domain comprises a co-stimulatory domain derived from one or more proteins selected from the group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, ligands of CD83, CD40, and MyD88.

In certain embodiments, the co-stimulatory domain is derived from 4-1BB.

In certain embodiments, the CAR comprises an intracellular signaling domain, wherein the intracellular signaling domain comprises an intracellular signaling domain derived from one or more proteins selected from the group consisting of CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP12, and domains containing at least one ITAM.

In certain embodiments, the intracellular signaling domain is derived from CD3ζ.

In certain embodiments, the iNKT cell is a CAR-iNKT cell, wherein the CAR comprises, in sequence, a CD8 signal peptide, a GPC3 antibody, a CD8 hinge region, a CD28 transmembrane domain, a 4-1BB co-stimulatory domain, a CD3ζ intracellular signaling domain, a GSG linker sequence, a P2A self-cleaving peptide, an IgGκ signal peptide, a PD-1 antibody, a V5 tag, (GGGGS)₄, and a targeting sequence. In certain embodiments, the amino acid sequence of the PD-1 antibody is as set forth in SEQ ID NO: 19, and the amino acid sequence of the targeting sequence is as set forth in SEQ ID NO: 8.

In certain embodiments, the iNKT cell is a CAR-iNKT cell, wherein the CAR comprises, in sequence, a CD8 signal peptide, an ROR1 antibody, a CD28 hinge region, a CD28 transmembrane domain, a 4-1BB co-stimulatory domain, a CD3ζ intracellular signaling domain, a GSG linker sequence, a P2A self-cleaving peptide, an IgGκ signal peptide, a PD-1 antibody, a V5 tag, (GGGGS)₄, and a targeting sequence. In certain embodiments, the amino acid sequence of the PD-1 antibody is as set forth in SEQ ID NO: 19, and the amino acid sequence of the targeting sequence is as set forth in SEQ ID NO: 8.

In certain embodiments, the CAR comprises a cytokine domain.

In certain embodiments, the CAR comprises a cytokine domain.

In certain embodiments, the cytokine domain:
(1) comprises one or more cytokines and/or functional fragments thereof;
(2) comprises one or more cytokine receptors and/or functional fragments thereof; and
(3) comprises a combination of (1) and (2).

In certain embodiments, the cytokine domain is expressed on the iNKT cell membrane.

In certain embodiments, the cytokine domain is secreted from the iNKT cell.

In certain embodiments, the cytokine is selected from one or more factors from the group consisting of IL-15, IL-10, IL-18, and IL-21.

In certain embodiments, the cytokine is IL-15.

In certain embodiments, the cytokine receptor is IL-15R or its alpha subunit (IL-15Rα).

In another aspect, the present application provides a nucleic acid molecule encoding a sequence comprising a nucleic acid sequence encoding a negative immune regulator gene and a nucleic acid sequence encoding a targeting sequence, wherein the negative immune regulator gene is selected from one or more genes from the group consisting of PD-1, Tim-3, CTLA-4, CD74, and TGF-βR.

In certain embodiments, the negative immune regulator gene is PD-1. In certain embodiments, the amino acid sequence of the PD-1 is as set forth in SEQ ID NO: 12 or SEQ ID NO: 19.

In certain embodiments, the targeting sequence is selected from the group consisting of an endoplasmic reticulum retention sequence, a Golgi retention sequence, an E3 ubiquitin ligase binding sequence, a proteasome targeting sequence, and a lysosomal targeting sequence.

In certain embodiments, the targeting sequence is an endoplasmic reticulum retention sequence.

In certain embodiments, the targeting sequence is a lysosomal targeting sequence.

In certain embodiments, the amino acid sequence of the endoplasmic reticulum retention sequence comprises one or more amino acid sequences selected from the group consisting of KDEL (SEQ ID NO: 9), KKMP (SEQ ID NO: 10), and AEKDEL (SEQ ID NO: 11).

In certain embodiments, the amino acid sequence of the lysosomal targeting sequence is as set forth in SEQ ID NO: 8.

In certain embodiments, the nucleic acid molecule comprises a nucleic acid sequence encoding an antigen-targeting moiety.

In certain embodiments, the antigen is selected from one or more antigens from the group consisting of GPC3, CLDN18.2, ROR1, and AXL.

In certain embodiments, the antigen is GPC3. In certain embodiments, the antigen is ROR1.

In certain embodiments, the nucleic acid molecule comprises a nucleic acid sequence encoding a promoter.

In certain embodiments, the promoter is selected from the group consisting of EF1α, CMV, MSCV, SSFV, and UbC.

In certain embodiments, the nucleic acid molecule comprises a nucleic acid sequence encoding a signal peptide.

In certain embodiments, the signal peptide is derived from the CD8 protein.

In certain embodiments, the signal peptide is derived from IL-2.

In certain embodiments, the nucleic acid molecule comprises a nucleic acid sequence encoding a hinge region, wherein the hinge region comprises a hinge region derived from one or more proteins selected from the group consisting of CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

In certain embodiments, the hinge region is derived from CD28. In certain embodiments, the hinge region is derived from CD8.

In certain embodiments, the nucleic acid molecule comprises a nucleic acid sequence encoding a transmembrane domain, wherein the transmembrane domain comprises a transmembrane domain derived from one or more proteins selected from the group consisting of CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

In certain embodiments, the transmembrane domain is derived from CD28.

In certain embodiments, the nucleic acid molecule comprises a nucleic acid sequence encoding a co-stimulatory domain, wherein the co-stimulatory domain comprises a co-stimulatory domain derived from one or more proteins selected from the group consisting of CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, ligands of CD83, CD40, and MyD88.

In certain embodiments, the co-stimulatory domain is an intracellular co-stimulatory domain derived from 4-1BB.

In certain embodiments, the nucleic acid molecule comprises a nucleic acid sequence encoding an intracellular signaling domain, wherein the intracellular signaling domain comprises an intracellular signaling domain derived from one or more proteins selected from the group consisting of CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP12, and domains containing at least one ITAM.

In certain embodiments, the intracellular signaling domain is derived from CD3ζ.

In certain embodiments, the targeting sequence is located upstream of the sequence of the negative immune regulator gene.

In certain embodiments, the targeting sequence is located downstream of the sequence of the negative immune regulator gene.

In certain embodiments, the nucleic acid molecule comprises a sequence encoding a cleavage peptide.

In certain embodiments, the cleavage peptide is selected from one or more cleavage peptides from the group consisting of P2A, T2A, F2A, E2A, BmCPV2A, IRES, and BmIFV2A.

In certain embodiments, the cleavage peptide is P2A.

In certain embodiments, the cleavage peptide P2A is linked to the GSG linker sequence.

In certain embodiments, the nucleic acid molecule encodes, in sequence, a CD8 signal peptide, a GPC3 antibody, a CD8 hinge region, a CD8 transmembrane domain, a 4-1BB co-stimulatory domain, a CD3ζ intracellular signaling domain, a GSG linker sequence, a P2A self-cleaving peptide, an IgGκ signal peptide, a PD-1 antibody, a V5 tag, (GGGGS)₄, and a targeting sequence. In certain embodiments, the amino acid sequence of the PD-1 antibody is as set forth in SEQ ID NO: 19, and the amino acid sequence of the targeting sequence is as set forth in SEQ ID NO: 8.

In certain embodiments, the nucleic acid molecule encodes, in sequence, a CD8 signal peptide, an ROR1 antibody, a CD28 hinge region, a CD8 transmembrane domain, a 4-1BB co-stimulatory domain, a CD3ζ intracellular signaling domain, a GSG linker sequence, a P2A self-cleaving peptide, an IgGκ signal peptide, a PD-1 antibody, a V5 tag, (GGGGS)₄, and a targeting sequence. In certain embodiments, the amino acid sequence of the PD-1 antibody is as set forth in SEQ ID NO: 19, and the amino acid sequence of the targeting sequence is as set forth in SEQ ID NO: 8.

In certain embodiments, the nucleic acid molecule comprises a nucleic acid sequence encoding a cytokine domain.

In certain embodiments, the nucleic acid sequence encoding the cytokine domain may be:
(1) a sequence capable of expressing one or more cytokines and/or functional fragments thereof;
(2) a sequence capable of expressing one or more cytokine receptors and/or functional fragments thereof; and
(3) A sequence capable of expressing the combination of (1) and (2).

In certain embodiments, the cytokine is selected from one or more of the following: IL-15, IL-10, IL-18, and IL-21.

In certain embodiments, the cytokine is IL-15.

In certain embodiments, the cytokine receptor is IL-15R or its alpha subunit (IL-15Rα).

In another aspect, the present application provides a plasmid comprising the nucleic acid molecule described herein.

In certain embodiments, the plasmid includes viral plasmids and bacterial plasmids.

In certain embodiments, the plasmid includes DNA plasmids and RNA plasmids.

In certain embodiments, the plasmid includes linear plasmids and circular plasmids.

In another aspect, the present application provides a pharmaceutical composition comprising the iNKT cells, the nucleic acid molecules, the plasmids described herein, and at least one pharmaceutically acceptable carrier.

In another aspect, the iNKT cells, nucleic acid molecules, plasmids, and pharmaceutical compositions described herein are useful in the manufacture of a medicament for the prevention, alleviation, and/or treatment of diseases and/or disorders.

Other aspects and advantages of the present application will be readily apparent to those skilled in the art from the detailed description below. Only exemplary embodiments of the present application are shown and described in the detailed description below. As will be recognized by those skilled in the art, the content of the present application enables those skilled in the art to make modifications to the specific embodiments disclosed herein without departing from the spirit and scope of the invention described herein. Accordingly, the description in the drawings and specification of the present application is merely exemplary and not intended to be limiting.

### Brief Description of the Drawings

The specific features of the invention described herein are set forth in the appended claims. The features and advantages of the invention described herein can be better understood by reference to the exemplary embodiments and drawings described in detail below. A brief description of the drawings is provided below:
Figure 1 shows detection of PD1 expression levels after co-culturing the PD1-knockout and non-knockout ROR1 CAR-iNKT cells described herein with tumor cells for three days.
Figure 2 shows validation of TGFβRII knockout in the ROR1 CAR-iNKT cells described herein.
Figures 3A, 3B, and 3C show comparisons of tumor cell killing after knockout of PD1 and TGFβRII as described herein.
Figure 4A shows detection of killing function of the PD1-knockout and non-knockout ROR1 CAR-iNKT cells described herein after secondary stimulation with tumor cells.
Figure 4B shows detection of survival rates of the PD1-knockout and non-knockout ROR1 CAR-iNKT cells described herein during in vitro co-culture.
Figure 5A shows a schematic diagram of the construction of GPC3-CAR and GPC3-CAR PD1KO described herein.
Figure 5B shows the expression efficiency on iNKT cells of the GPC3-CAR PD1KO constructs with and without PD1 knockout as described herein.
Figure 6 shows detection of tumor inhibition efficacy of the GPC3-CAR PD1 KO described herein.
Figure 7A shows a schematic diagram of the CAR construct expressing IL-15 in the PD1-knockout and non-knockout GPC3 CAR-iNKT cells described herein.
Figure 7B shows detection results of expression of the IL-15-enhanced CAR in PBMCs as described herein.
Figure 7C shows detection results of IL-15 secretion levels in the supernatant 72 hours after transduction as described herein.
Figure 7D shows detection of expression levels of the PD1-knockout and non-knockout GPC3 CAR-iNKT cells described herein.
Figure 7E shows detection of IL-15 secretion levels in the supernatant 72 hours after transduction as described herein.
Figure 8A shows detection of killing function against Hep3B and Huh7 cells expressing GPC3 by secreted or membrane receptor-anchored IL-15 as described herein.
Figure 8B shows that secreted or membrane receptor-anchored IL-15 as described herein enhances IFN-γ secretion.
Figure 9 shows a schematic diagram illustrating how the tri-specific CAR architecture described herein reduces PD1 expression in tumor killing.

### Detailed Description

The following specific examples illustrate embodiments of the invention described herein; persons skilled in the art can readily understand other advantages and effects of the invention from the disclosure herein.

### Definition of Terms

In the present application, the term "iNKT cells" generally refers to a specialized subset of T cells involved in innate immunity, characterized by an invariant TCRα receptor. iNKT cells can recognize antigens in the presence of CD1d molecules and specifically recognize α-GalCer; iNKT cells can be labeled using CD1d/α-GalCer tetramers. iNKT cells can differentiate into NKT1, NKT2, and NKT17 subsets, analogous to Th1, Th2, and Th17 subsets. iNKT cells contain large amounts of cytotoxic cytokines (e.g., IL-4, IL-10, IFN-γ, etc.), significantly higher than those in NK cells and T cells; upon stimulation, iNKT cells rapidly release cytokines to kill tumor cells.

In the present application, the term "membrane protein intracellular retention technology" generally refers to a technique that inhibits gene expression by using targeting sequences to carry specific proteins.

In the present application, the term "lack" generally means that the level (e.g., protein expression level) is essentially not expressed compared to the original, or cannot exert its original function.

In the present application, the term "inhibit expression" generally means that the level (e.g., protein expression level) is reduced compared to the original. For example, the degree of reduction can be determined by P-values, such as P<0.05, P<0.04, P<0.03, P<0.02, P<0.01, P<0.005, or P<0.001. For example, the inhibition of expression may represent a reduction of 5% or more, 10% or more, 20% or more, 30% or more, 40% or more, 50% or more, 60% or more, 70% or more, 80% or more, 90% or more, or 100% or more compared to the original.

In the present application, the term "chimeric antigen receptor" commonly referred to as "CAR", generally refers to a fusion protein comprising an extracellular domain capable of binding an antigen and at least one intracellular domain. In the present application, the CAR may comprise an intracellular domain, which includes a signaling domain and/or a co-stimulatory domain. In the present application, a set of polypeptides of the CAR may reside on the same polypeptide chain (e.g., comprising a chimeric fusion protein) or may be discontinuous, for example, located on different polypeptide chains. In the present application, signals involved in induction can be transduced into the cytoplasm of T cells via CD3 and the ζ chain. In the present application, the intracellular domain may comprise a primary signaling binding domain (e.g., the primary signaling domain of CD3-zeta (ζ)). In one aspect, the cytoplasmic signaling domain may further comprise one or more co-stimulatory domains derived from at least one co-stimulatory molecule. For example, the co-stimulatory domain may be 4-1BB (i.e., CD137), CD27, ICOS, and/or CD28. In the present application, the CAR may comprise a chimeric fusion protein, for example, containing an optional leader sequence at the amino terminus (N-ter). Wherein the leader sequence optionally cleaves the antigen-binding domain (e.g., ScFv) during cellular processing and localizes the CAR to the cell membrane.

In the present application, the term "negative immune regulator genes" generally refers to genes that inhibit cellular immune responses through negative regulation. For example, genes encoding immune checkpoint-related proteins. In the present application, negative immune regulator genes include, but are not limited to, PD-1, Tim-3, CTLA-4, CD74, and TGF-βR.

In the present application, the term "targeting sequence" generally refers to a sequence that directs a polypeptide to a subcellular structure. For example, endoplasmic reticulum retention sequence, Golgi retention sequence, E3 ubiquitin ligase binding sequence, proteasome targeting sequence, or lysosomal targeting sequence. In the present application, the targeting sequence may be directly linked to the adjacent polypeptide or connected via a linker sequence. In the present application, the targeting sequence may be located at the N-terminus or C-terminus of the polypeptide.

In the present application, the term "endoplasmic reticulum retention sequence" generally refers to a short peptide signal sequence that has a corresponding receptor on the Golgi membrane and can be transported back to the endoplasmic reticulum via retrograde vesicles. Endoplasmic reticulum retention sequences suitable for the present invention may be those well known in the art. In the present application, the endoplasmic reticulum retention sequence may be located at the C-terminus or N-terminus of the adjacent polypeptide. In the present application, endoplasmic reticulum retention sequences include, but are not limited to, KDEL (SEQ ID NO: 9), KKMP (SEQ ID NO: 10), and AEKDEL (SEQ ID NO: 11).

In the present application, the term "Golgi retention sequence" generally refers to a short peptide signal sequence that targets a corresponding receptor on the Golgi membrane. In the present application, Golgi retention sequences include, but are not limited to, KDEL (SEQ ID NO: 9), KKMP (SEQ ID NO: 10), and AEKDEL (SEQ ID NO: 11).

In the present application, the term "E3 ubiquitin ligase binding sequence" generally refers to a short peptide signal sequence that can target the constant region (Fc) gene of human immunoglobulin G (IgG) (e.g., IgG1, IgG2, or IgG4).

In the present application, the term "proteasome targeting sequence" generally refers to a short peptide signal sequence implemented by linking an antibody sequence to a sequence containing an E3 ubiquitin ligase targeting domain and co-expressing the nucleic acid sequence encoding the E3 ubiquitin ligase protein. The E3 ubiquitin ligase protein binds with high affinity to the Fc domain of antibodies and can recruit the ubiquitin-proteasome complex to degrade molecules (e.g., proteins and peptides) bound to the antibody. In the present application, proteasome targeting sequences include, but are not limited to, the PEST motif (SEQ ID NO: 22).

In the present application, the term "lysosomal targeting sequence" generally refers to a short peptide signal sequence. In the present application, lysosomal targeting sequences include, but are not limited to, the KFERQ motif (SEQ ID NO: 20). In the present application, lysosomal targeting sequences include, but are not limited to, the SFHDDSDEDLLHI motif (SEQ ID NO: 8).

In the present application, the term "cytokine domain" generally refers to a domain capable of expressing a cytokine and/or its functional fragment, or capable of expressing one or more cytokine receptors and/or their functional fragments, or capable of simultaneously expressing a cytokine and/or its functional fragment along with one or more cytokine receptors and/or their functional fragments.

In the present application, the term "targeting moiety" generally refers to a molecule or fragment that can specifically recognize and bind to a cell surface marker. Targeting moieties include, but are not limited to, antibodies or fragments thereof, peptides, hormones, growth factors, cytokines, and any other natural or non-natural ligands.

In the present application, the term "signal peptide" generally refers to a prepeptide present as an N-terminal peptide on the precursor form of a protein. The function of the signal peptide can be to facilitate translocation of the expressed polypeptide linked to the endoplasmic reticulum. The signal peptide is typically cleaved during this process. The signal peptide cleaved from the protein precursor may be heterologous or homologous to the organism used to produce the polypeptide.

In the present application, the term "hinge region" generally refers to the connecting region between the antigen-binding region and the Fc receptor (FcR)-binding region of immune cells. For example, the hinge region may be the region between the CH1 and CH2 domains of the immunoglobulin heavy chain.

In the present application, the term "transmembrane domain" generally refers to the region connecting the extracellular antigen-binding domain and the intracellular signaling domain, typically composed of dimeric membrane proteins, including mainly CD3ζ, CD4, CD8, CD28, etc., which anchor the CAR structure to the T-cell membrane. Different designs of the transmembrane domain can affect the expression of the introduced CAR gene.

In the present application, the term "co-stimulatory domain" generally refers to the domain of the CAR that traverses the cell membrane and is connected to the intracellular signaling domain, serving to transmit signals.

In the present application, the term "intracellular signaling domain" generally refers to a domain located inside the cell capable of transducing signals. In the present application, the intracellular signaling domain can transduce signals into the cell interior. For example, the intracellular signaling domain is the intracellular signaling domain of the chimeric antigen receptor.

In the present application, the term "promoter" generally refers to a DNA sequence that regulates the expression of a selected DNA sequence operably linked to the promoter, thereby influencing the expression of the selected DNA sequence in the cell.

In the present application, the term "cleavage peptide" generally refers to a class of peptides capable of mediating protein cleavage. For example, the cleavage peptide can achieve protein cleavage via ribosomal skipping rather than proteolytic hydrolysis. For example, the cleavage peptide may be a 2A cleavage peptide, which may include T2A, F2A, P2A, and/or E2A. In the present application, the cleavage peptide may also be linked to a linker sequence, which may be GSG.

In the present application, the term "single-chain antibody" (ScFv) generally refers to an antibody formed by linking the heavy chain variable region and the light chain variable region via a linker.

In the present application, the term "VHH" generally refers to an antibody comprising the variable antigen-binding domain of a heavy-chain antibody. VHH may also be referred to as nanobody (Nb) and/or single-domain antibody.

In the present application, the term "plasmid" generally refers to any molecule capable of transferring encoded information into a cell. For example, a plasmid may be a linear or circular autonomously replicating sequence, genomic integration sequence, phage, or nucleotide sequence of single-stranded or doublestranded DNA or RNA derived from any source.

In the present application, the term "pharmaceutical composition" generally refers to a formulation in a form that allows the biological activity of the active ingredient to be effective and does not contain additional components that are unacceptably toxic to the subject to whom the formulation is to be administered.

In the present application, the term "pharmaceutically acceptable carrier" may include buffers, antioxidants, preservatives, low molecular weight polypeptides, proteins, hydrophilic polymers, amino acids, sugars, chelating agents, counterions, metal complexes, and/or nonionic surfactants, among others.

In the present application, the term "about" generally refers to a variation within a range of 0.5%-10% above or below the specified value, for example, within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present application, the term "diagnosis" generally refers to detecting a disease or disorder, or determining the status or severity of a disease or disorder, and also includes detecting causative factors of a disease or disorder, assessing the therapeutic efficacy of drug treatment, or predicting the response pattern to drug therapy or xenobiotics. The diagnostic methods described in the present application may be used independently or in combination with other diagnostic and/or staging methods for specific diseases or disorders known in the medical field.

In the present application, the term "prevention" generally refers to methods intended to prevent, inhibit, or reduce the likelihood of the occurrence or recurrence of a disease or condition (e.g., cancer). It also refers to delaying the onset or recurrence of a disease or condition or delaying the appearance or recurrence of symptoms of a disease or condition. The term "prevention" and similar terms also encompass reducing the intensity, impact, symptoms, and/or burden of a disease or condition prior to its onset or recurrence.

In the present application, the term "treatment" generally refers to any beneficial or desirable effect on the symptoms or lesions of a disease or pathological condition, and may even include a minimal reduction in one or more measurable markers of the treated disease or condition (e.g., cancer). Treatment may optionally involve reduction or amelioration of the symptoms of a disease or condition, or delay in the progression of the disease or condition. The term "treatment" does not necessarily indicate complete eradication or cure of the disease or condition or its associated symptoms.

In the present application, the term "proliferation" generally refers to the process by which an initial cell generates multiple cellular individuals through division. The multiple cellular individuals may be cells of the same type or of different types. The initial cell used for proliferation need not be identical to the cells produced by proliferation. For example, proliferating cells may arise from the growth and differentiation of an initial cellular population.

In the present application, the term "differentiation" generally refers to the process by which a nonspecific or less specific cell acquires characteristics of a specific cell type. Differentiated or differentiation-inducible cells are cells occupying a more specific position within a cell lineage.

In the present application, the term "ex vivo" generally refers to procedures involving manipulation of cells, tissues, and/or organs that have been removed from a living organism. In certain embodiments, the cells, tissues, and/or organs may be reintroduced into the original organism or transferred into another organism by certain methods.

In the present application, the term "in vitro" generally refers to removing or releasing a part of an organism from the organism.

In the present application, the term "comprising" generally has the meaning of including, encompassing, containing, or incorporating. In certain contexts, it may also mean "being," or "consisting of."

In the present application, the term "and/or" should be understood to mean any one, two, several, or any combination thereof among the listed alternatives.

In the present application, the term "about" generally refers to a variation within a range of 0.5%-10% above or below the specified value, for example, within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### Detailed Description of the Invention

### modified iNKT cell

In one aspect, the present application provides a modified iNKT cell that, compared to an unmodified iNKT cell, lacks or inhibits expression of one or more negative immune regulator genes selected from the group consisting of: PD-1, Tim-3, CTLA-4, CD74, and TGF-βR, wherein the lack or inhibition of expression of the negative immune regulator genes are achieved via membrane protein intracellular retention technology.

In the present application, the negative immune regulator gene may be a gene associated with T-cell immune checkpoints, a gene that inhibits T-cell proliferation, a gene that inhibits tumor expression, or a gene that exerts tumor suppressor effects by inducing cell cycle arrest and promoting apoptosis. For example, the negative immune regulator gene may be PD-1. For example. The amino acid sequence of the negative immune regulator gene may be as set forth in SEQ ID NO: 12 or SEQ ID NO: 19.

In the present application, the modified iNKT cell may express a chimeric antigen receptor (CAR).

In the present application, the CAR may comprise a targeting sequence that directs the polypeptide to a subcellular structure.

For example, the subcellular structure includes, but is not limited to, the Golgi apparatus or endoplasmic reticulum, proteasome, cell membrane, or lysosome.

For example, the targeting sequence may be selected from an endoplasmic reticulum retention sequence, a Golgi retention sequence, an E3 ubiquitin ligase binding sequence, a proteasome targeting sequence, and a lysosomal targeting sequence.

For example, the targeting sequence may be located at the N-terminus or C-terminus of the polypeptide.

For example, the targeting sequence may be directly linked to the adjacent polypeptide or connected via a linker sequence well known in the art. For example, a linker sequence comprising G and S.

In the present application, the endoplasmic reticulum retention sequence is a short peptide signal sequence. The endoplasmic reticulum retention sequence has a corresponding receptor on the Golgi membrane and can be transported back to the endoplasmic reticulum via retrograde vesicles.

For example, the endoplasmic reticulum retention sequence may be located at the C-terminus, the N-terminus, or both the C- and N-termini of the adjacent polypeptide.

For example, the endoplasmic reticulum retention sequence may be an endoplasmic reticulum retention sequence well known in the art. For example, the "endoplasmic reticulum retention sequence" or "Golgi retention sequence" includes, but is not limited to, KDEL (SEQ ID NO: 9), KKMP (SEQ ID NO: 10), and AEKDEL (SEQ ID NO: 11). For example, the amino acid sequence of the endoplasmic reticulum retention sequence is as set forth in SEQ ID NO: 8. For example, the amino acid sequence of the lysosomal targeting sequence is as set forth in SEQ ID NO: 8.

In the present application, the proteasome targeting sequence can be achieved by linking an antibody sequence to a nucleic acid sequence encoding an E3 ubiquitin ligase targeting domain and co-expressing it with a nucleic acid sequence encoding the E3 ubiquitin ligase protein. The E3 ubiquitin ligase protein binds with high affinity to the Fc domain of antibodies and can recruit the ubiquitin-proteasome complex to degrade molecules bound to the antibody. For example, the proteasome targeting sequence includes, but is not limited to, the PEST (SEQ ID NO: 22) motif.

In the present application, the E3 ubiquitin ligase protein targeting domain sequence encodes an amino acid sequence selected from the constant region (Fc) genes of human immunoglobulin G (IgG) (e.g., IgG1, IgG2, or IgG4) and is used to form a fusion protein comprising an antibody and the Fc domain. For example, the lysosomal targeting sequence includes, but is not limited to, the KFERQ (SEQ ID NO: 20) motif.

In the present application, the CAR comprises a targeting moiety that specifically binds to an antigen. In the present application, the targeting moiety may include an antibody or an antigen-binding fragment. The antigen-binding fragment may be selected from the group consisting of: Fab, Fab', F(ab)₂, F(ab')₂, Fv, VHH, and scFv fragments. For example, the targeting moiety may be an scFv. For example, the targeting moiety may be a VHH.

For example, the antigen may be selected from one or more antigens from the group consisting of: GPC3, CLDN18.2, ROR1, and AXL. For example, the antigen may be GPC3. For example, the antigen may be CLDN18.2. For example, the antigen may be ROR1. For example, the antigen may be AXL. For example, the antigen may comprise the amino acid sequence shown in SEQ ID NO: 1 or an amino acid sequence having at least 80% homology thereto. For example, the antigen may comprise the amino acid sequence shown in SEQ ID NO: 15 or an amino acid sequence having at least 80% homology thereto.

In the present application, the CAR comprises a signal peptide. For example, the signal peptide may be derived from the CD8 protein. For example, the signal peptide may be derived from the CD8 protein. For example, the signal peptide may be derived from IL-2. For example, the signal peptide may comprise the amino acid sequence shown in SEQ ID NO: 2.

In the present application, the CAR comprises a hinge region, which may be any oligopeptide or polypeptide connecting the transmembrane domain to the extracellular ligand-binding domain, providing greater flexibility and accessibility to the extracellular ligand-binding domain; the hinge region may be a synthetic sequence corresponding to a naturally occurring hinge sequence, or it may be a fully synthetic hinge sequence.

For example, the hinge region comprises a hinge region derived from one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT. For example, the hinge region may be derived from CD28. For example, the hinge region may comprise the amino acid sequence shown in SEQ ID NO: 3.

In the present application, the CAR comprises a transmembrane domain that traverses the cell membrane, anchors the CAR to the cell surface, and connects the extracellular domain to the intracellular signaling domain, thereby influencing CAR expression on the cell surface.

For example, the transmembrane domain comprises a transmembrane domain derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM. For example, the transmembrane domain may be derived from CD28. For example, the transmembrane domain may comprise the amino acid sequence shown in SEQ ID NO: 4.

In the present application, the CAR comprises a co-stimulatory domain derived from the intracellular signaling domain of a co-stimulatory protein, which can enhance cytokine production, proliferation, cytotoxicity, and/or persistence in vivo.

For example, the co-stimulatory domain comprises a co-stimulatory domain derived from one or more proteins selected from the group consisting of: CD28, 4-1BB, CD27, CD2, CD7, CD8, OX40, CD226, DR3, SLAM, CD5, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, ligands of CD83, CD40, and MyD88. For example, the co-stimulatory domain is derived from 4-1BB. For example, the co-stimulatory domain may comprise the amino acid sequence shown in SEQ ID NO: 5.

In the present application, the CAR comprises an intracellular signaling domain capable of transmitting signals into the cell.

For example, the intracellular signaling domain comprises an intracellular signaling domain derived from one or more proteins selected from the group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP12, and domains containing at least one ITAM. For example, the intracellular signaling domain is derived from CD3ζ. For example, the intracellular signaling domain may comprise the amino acid sequence shown in SEQ ID NO: 6.

In the present application, the CAR comprises a cytokine domain, which may:
(1) comprise one or more cytokines and/or functional fragments thereof;
(2) comprise one or more cytokine receptors and/or functional fragments thereof; and
(3) comprise a combination of (1) and (2).

In the present application, the cytokine domain is expressed on the iNKT cell membrane. In the present application, it is secreted from iNKT cells.

In the present application, the cytokines include interleukins, interferons, and/or tumor necrosis factors.

For example, the cytokine is selected from one or more factors from the group consisting of: IL-15, IL-10, IL-18, and IL-21. For example, the cytokine comprises IL-2, IL-4, IL-6, IL-7, IL-12, TNF-α, and/or IFNγ. For example, the cytokine is IL-15. For example, the cytokine receptor is IL-15R or its alpha subunit (IL-15Rα). For example, the cytokine domain may comprise the amino acid sequence shown in SEQ ID NO: 17 or SEQ ID NO: 18.

In the present application, the iNKT cell may be a CAR-iNKT cell, wherein the CAR consists of, in sequence: a CD8 signal peptide, a GPC3 antibody, a CD8 hinge region, a CD8 transmembrane domain, a 4-1BB co-stimulatory domain, a CD3ζ intracellular signaling domain, a GSG linker sequence, a P2A self-cleaving peptide, an IgGκ signal peptide, a PD-1 antibody, a V5 tag, (GGGS)₄, and a targeting sequence.

For example, the amino acid sequence of the CD8 signal peptide is as set forth in SEQ ID NO: 2, the GPC3 antibody as set forth in SEQ ID NO: 15, the CD8 hinge region as set forth in SEQ ID NO: 16, the CD28 transmembrane domain as set forth in SEQ ID NO: 4, the 4-1BB co-stimulatory domain as set forth in SEQ ID NO: 5, the CD3ζ intracellular signaling domain as set forth in SEQ ID NO: 6, the GSG linker sequence and P2A self-cleaving peptide as set forth in SEQ ID NO: 7, the IgGκ signal peptide as set forth in SEQ ID NO: 14, the PD-1 antibody as set forth in SEQ ID NO: 19, the V5 tag as set forth in SEQ ID NO: 13, (GGGGS)₄ as set forth in SEQ ID NO: 21, and the targeting sequence as set forth in SEQ ID NO: 8.

For example, the amino acid sequence of the CD8 signal peptide is as set forth in SEQ ID NO: 2, the ROR1 antibody as set forth in SEQ ID NO: 1, the CD28 hinge region as set forth in SEQ ID NO: 3, the CD28 transmembrane domain as set forth in SEQ ID NO: 4, the 4-1BB co-stimulatory domain as set forth in SEQ ID NO: 5, the CD3ζ intracellular signaling domain as set forth in SEQ ID NO: 6, the GSG linker sequence and P2A self-cleaving peptide as set forth in SEQ ID NO: 7, the IgGκ signal peptide as set forth in SEQ ID NO: 14, the PD-1 antibody as set forth in SEQ ID NO: 19, the V5 tag as set forth in SEQ ID NO: 13, (GGGS)₄ as set forth in SEQ ID NO: 21, and the targeting sequence as set forth in SEQ ID NO: 8.

For example, the amino acid sequence of the CD8 signal peptide is as set forth in SEQ ID NO: 2, the GPC3 antibody as set forth in SEQ ID NO: 15, the CD8 hinge region as set forth in SEQ ID NO: 16, the CD28 transmembrane domain as set forth in SEQ ID NO: 4, the 4-1BB co-stimulatory domain as set forth in SEQ ID NO: 5, the CD3ζ intracellular signaling domain as set forth in SEQ ID NO: 6, the GSG linker sequence and P2A self-cleaving peptide as set forth in SEQ ID NO: 7, the IgGκ signal peptide as set forth in SEQ ID NO: 14, the PD-1 antibody as set forth in SEQ ID NO: 12, the V5 tag as set forth in SEQ ID NO: 13, (GGGGS)₄ as set forth in SEQ ID NO: 21, and the targeting sequence as set forth in SEQ ID NO: 11.

For example, the amino acid sequence of the CD8 signal peptide is as set forth in SEQ ID NO: 2, the ROR1 antibody as set forth in SEQ ID NO: 1, the CD28 hinge region as set forth in SEQ ID NO: 3, the CD28 transmembrane domain as set forth in SEQ ID NO: 4, the 4-1BB co-stimulatory domain as set forth in SEQ ID NO: 5, the CD3ζ intracellular signaling domain as set forth in SEQ ID NO: 6, the GSG linker sequence and P2A self-cleaving peptide as set forth in SEQ ID NO: 7, the IgGκ signal peptide as set forth in SEQ ID NO: 14, the PD-1 antibody as set forth in SEQ ID NO: 12, the V5 tag as set forth in SEQ ID NO: 13, (GGGS)₄ as set forth in SEQ ID NO: 21, and the targeting sequence as set forth in SEQ ID NO: 11.

### nucleic acid molecule

In another aspect, the present application provides a nucleic acid molecule encoding the CAR described herein.

In the present application, the nucleic acid molecule comprises a nucleic acid sequence encoding a targeting sequence, wherein the targeting sequence is selected from the group consisting of an endoplasmic reticulum retention sequence, a Golgi retention sequence, an E3 ubiquitin ligase binding sequence, a proteasome targeting sequence, and a lysosomal targeting sequence.

For example, the targeting sequence is an endoplasmic reticulum retention sequence. For example, the amino acid sequence of the endoplasmic reticulum retention sequence comprises one or more amino acid sequences selected from the group consisting of: KDEL (SEQ ID NO: 9), KKMP (SEQ ID NO: 10), and AEKDEL (SEQ ID NO: 11). For example, the targeting sequence is a lysosomal targeting sequence. For example, the amino acid sequence of the lysosomal targeting sequence is as set forth in SEQ ID NO: 8.

In the present application, the nucleic acid molecule comprises a nucleic acid sequence encoding a negative immune regulator gene, wherein the gene is selected from one or more genes from the group consisting of: PD-1, Tim-3, CTLA-4, CD74, and TGF-βR.

In the present application, the nucleic acid molecule comprises a nucleic acid sequence encoding a target antigen.

For example, the antigen is selected from one or more antigens from the group consisting of: GPC3, CLDN18.2, ROR1, and AXL. For example, the antigen is GPC3. For example, the antigen is CLDN18.2. For example, the antigen is ROR1. For example, the antigen is AXL.

In the present application, the nucleic acid molecule comprises a nucleic acid sequence encoding a promoter, wherein the promoter sequence is typically operably linked to the coding sequence of the protein to be expressed. The promoter may be any nucleic acid sequence exhibiting transcriptional activity in the selected host cell, including mutated, truncated, and hybrid promoters, and may be derived from a gene encoding an extracellular or intracellular polypeptide homologous or heterologous to the host cell.

For example, the promoter is selected from the group consisting of: EF1α, CMV, MSCV, SSFV, and UbC.

In the present application, the nucleic acid molecule comprises a nucleic acid sequence encoding a signal peptide.

For example, the signal peptide is a signal peptide derived from the CD8 protein. For example, the signal peptide is a signal peptide derived from the IL-2 protein.

In the present application, the nucleic acid molecule comprises a nucleic acid sequence encoding a hinge region, wherein the hinge region comprises a hinge region derived from one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

For example, the hinge region is a hinge region derived from CD28. For example, the hinge region is a hinge region derived from CD8.

In the present application, the nucleic acid molecule comprises a nucleic acid sequence encoding a transmembrane domain, wherein the transmembrane domain comprises a transmembrane domain derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

For example, the transmembrane domain is a transmembrane domain derived from CD28.

In the present application, the nucleic acid molecule comprises a nucleic acid sequence encoding a co-stimulatory domain, wherein the co-stimulatory domain comprises a co-stimulatory domain derived from one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD83 ligand, CD40, and MyD88.

For example, the co-stimulatory domain is an intracellular co-stimulatory domain derived from 4-1BB.

In the present application, the nucleic acid molecule comprises a nucleic acid sequence encoding an intracellular signaling domain, wherein the intracellular signaling domain comprises an intracellular signaling domain derived from one or more proteins selected from the group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP-12, and a domain comprising at least one ITAM.

For example, the intracellular signaling domain is an intracellular signaling domain derived from CD3ζ.

In the present application, the nucleic acid molecule comprises a nucleic acid sequence encoding a cytokine domain, wherein the nucleic acid sequence encoding the cytokine domain may be:
(1) a sequence capable of expressing one or more cytokines and/or functional fragments thereof;
(2) a sequence capable of expressing one or more cytokine receptors and/or functional fragments thereof; and
(3) a sequence capable of expressing a combination of (1) and (2).

For example, the cytokine is selected from one or more factors from the group consisting of: IL-15, IL-10, IL-18, and IL-21. For example, the cytokine is IL-15. For example, the cytokine receptor is IL-15R or its alpha subunit (IL-15Rα).

In the present application, in the nucleic acid molecule, the targeting sequence may be located upstream of the sequence encoding the target antigen.

In the present application, in the nucleic acid molecule, the targeting sequence may be located downstream of the sequence encoding the target antigen.

In the present application, in the nucleic acid molecule, the targeting sequence may be located both upstream and downstream of the sequence encoding the target antigen.

In the present application, the nucleic acid molecule comprises a sequence encoding a cleavage peptide, wherein the cleavage peptide is selected from one or more cleavage peptides from the group consisting of: P2A, T2A, F2A, E2A, BmCPV2A, IRES, and BmIFV2A.

For example, the cleavage peptide is P2A. For example, the cleavage peptide P2A is linked to the linker sequence GSG (SEQ ID NO: 7).

For example, the nucleic acid molecule may sequentially encode a CD8 signal peptide, a GPC3 antibody, a CD8 hinge region, a CD28 transmembrane domain, a 4-1BB co-stimulatory domain, a CD3ζ intracellular signaling domain, a GSG linker sequence, a P2A self-cleaving peptide, an IgGκ signal peptide, a PD-1 antibody, a V5 tag, (GGGGS)₄, and a targeting sequence.

For example, the nucleic acid molecule may sequentially encode a CD8 signal peptide, a ROR1 antibody, a CD28 hinge region, a CD28 transmembrane domain, a 4-1BB co-stimulatory domain, a CD3ζ intracellular signaling domain, a GSG linker sequence, a P2A self-cleaving peptide, an IgGκ signal peptide, a PD-1 antibody, a V5 tag, (GGGGS)₄, and a targeting sequence.

In the present application, the nucleic acid sequence of the nucleic acid molecule may be as illustrated in the examples and figures of the present application.

In the present application, the nucleic acid sequence of the nucleic acid molecule may be further modified according to the description of modified iNKT cells set forth in the detailed description of the present application.

### plasmid

In another aspect, the present application provides a plasmid comprising the nucleic acid molecule described herein.

In the present application, the plasmid includes viral plasmids and bacterial plasmids.

In the present application, the plasmid includes DNA plasmids and RNA plasmids.

In the present application, the plasmid includes linear plasmids and circular plasmids.

In the present application, the plasmid may be natural or artificially modified.

### pharmaceutical composition

In another aspect, the present application provides a pharmaceutical composition comprising the iNKT cells described herein, the nucleic acid molecule described herein, and/or the plasmid described herein, and at least one pharmaceutically acceptable carrier.

In the present application, the term "pharmaceutically acceptable carrier" refers to a carrier, diluent, and/or excipient that is pharmacologically and/or physiologically compatible with the subject and the pharmaceutically active ingredient, including but not limited to: pH adjusters, surfactants, carbohydrates, adjuvants, antioxidants, chelating agents, ionic strength enhancers, and preservatives.

### use

In another aspect, the present application provides the use of the composition described herein in the manufacture of a medicament for the prevention, alleviation, and/or treatment of a disease and/or disorder.

In another aspect, the present application further provides a method of preventing and/or treating a disease, comprising administering to a subject in need the modified iNKT cells described herein.

For example, the administered modified iNKT cells may be formulated pharmaceutically by any conventional method. For example, the active ingredient may be mixed or diluted with a carrier, excipient, or diluent. Examples of suitable carriers, excipients, or diluents include lactose, dextrose, sucrose, sorbitol, mannitol, glycine, polyethylene glycol, starch, gum arabic, alginic acid, gelatin, calcium phosphate, calcium silicate, cellulose, methylcellulose, microcrystalline cellulose, polyvinylpyrrolidone, water, methyl paraben, propyl paraben, talc, magnesium stearate, and mineral oil. The formulation may additionally comprise fillers, anti-caking agents, lubricants, wetting agents, flavoring agents, emulsifiers, preservatives, etc. The composition of the present invention may be formulated by any known method in the art to provide rapid, sustained, or delayed release of the active ingredient upon administration to a patient.

In the present application, administration of the cells may be performed via injection (e.g., intramuscular, intravenous, intraperitoneal, subcutaneous), or by other methods such as infusion, to ensure delivery of the cells in an active form into the bloodstream. The cells may also be administered via intratumoral, peritumoral, intralesional, or perilesional routes to exert local and systemic therapeutic effects. For example, administration may be performed via local or intravenous injection.

In the present application, the administered dose of the cells may be a single dose or multiple doses. For example, the actual administered amount of the cells may be determined based on various relevant factors, such as the type of disease; route of administration; age, sex, and/or weight of the patient; and severity of the patient's symptoms.

Without being bound by any theory, the examples below are provided solely for illustrative purposes regarding the preparation methods and uses of the present application and are not intended to limit the scope of the invention disclosed herein.

### Examples

| Name | Supplier | Catalog Number |
|---|---|---|
| DMEM medium | Thermo Fisher | 11965118 |
| IMDM medium | Thermo Fisher | 12440061 |
| FBS serum | Gibco | 10500064 |
| PENICILLIN STREPTOMYCIN SOL Penicillin/Streptomycin | Thermo Fisher | 15140122 |
| ELISA MAX^{™} Deluxe Set Human IL-15 Kit | Biolegend | 435104 |
| ELISA MAX^{™} Standard Set Human IL-2 Kit | Biolegend | 431801 |
| CD3/28 magnetic beads | ThermoFisher | 11452D |
| Ficoll-Paque^{™} PLUS | Cytiva | 17144003 |
| PE-labeled ROR1 protein | Acro Biosystems | RO1-HP2E3 |
| PE-labeled GPC3 protein | Acro Biosystems | GP3-HP2E3 |
| FITC-labeled GPC3 protein | Acro Biosystems | GP3-HF2H1 |
| BV421-labeled PD1 antibody | Biolegend | 367422 |
| BV421-labeled TGFβRII antibody | Biolegend | 399710 |
| Pierce^{™} Protein Concentrator | Thermo | 88515 |
| Human TGFβ cytokine | Peprotech | 100-21-50UG |

### Example 1 Preparation of Cells

### Cell Culture

HEK293T, MEC1, Hep3B, Huh7, and MDA-MB-231 cell lines were obtained from ATCC, USA. The MEC1-ROR1 cell line was generated internally. HEK293T, Hep3B, and Huh7 cell lines were maintained in complete medium (DMEM containing 10% heat-inactivated FBS, 100 U/mL penicillin/streptomycin, and 2 mM L-glutamine). MDA-MB-231, MEC1, and MEC1-ROR1 cell lines were maintained in complete medium (IMDM containing 10% heat-inactivated FBS, 100 U/mL penicillin/streptomycin, and 2 mM L-glutamine). PBMCs were isolated from whole blood of healthy donors using Ficoll-Paque and aliquoted at a concentration of 2×10⁷ cells/mL in 1 mL samples, cryopreserved in liquid nitrogen tanks in medium consisting of heat-inactivated FBS supplemented with 10% DMSO (vol/vol).

### Construct Cloning

All relevant gene constructs were synthesized by GeneWiz. The gene construct encoding PD1 A2 VHH-PEBL-GSGP2A-ROR1 CAR was inserted into the pALD expression plasmid via SaII and SpeI cloning sites. To retain the PD1 protein intracellularly, an IgGk signal peptide (IgGKSP) was added to the N-terminus of PD1 A2 VHH (SEQ ID NO:19), and a PEBL sequence (LSM: SEQ ID NO:8) was appended to its C-terminus.

### Lentivirus Preparation

All lentiviruses were prepared using HEK293T cells. Freshly resuspended HEK293T cells were washed with ice-cold PBS buffer and seeded into 10 cm culture dishes at a confluency of 90-95%. The HEK293T cells were transiently transfected with a mixture of lentiviral packaging plasmids, transfer plasmids, and Lipofectamine-2000/3000. After 48 hours of culture, the supernatant was collected, filtered through a 0.45 µm filter, aliquoted at 1 mL per tube, and stored at -80°C.

### T Cell Activation and Transduction

To transduce PBMCs with lentiviruses encoding the respective constructs, cryopreserved PBMCs were thawed, plated into 24-well plates, and activated for 24 hours with CD3/CD28-conjugated magnetic beads and IL-2 (500 IU/mL). Activated PBMCs were distributed into multiple wells of a 24-well plate, followed by addition of the corresponding lentivirus and polybrene (8 µg/mL). The plate was centrifuged at 2000 × g for 2 hours in a benchtop centrifuge and then incubated at 37°C in a 5% CO₂ incubator. Transduced cells were further cultured for 48 hours in X-VIVO-15 medium supplemented with IL-2 (500 IU/mL) prior to subsequent assays and analyses.

### Flow Cytometry

Flow cytometric analysis was performed using a flow cytometer (Beckman Coulter) in plate mode, and data were analyzed using FlowJo software. Cells were washed once with FACS buffer (PBS containing 0.5% BSA, 0.1% NaN3, 2 mM EDTA, pH 7.0), resuspended at 5×10⁷ cells/mL, and kept on ice prior to staining. Fluorophore-conjugated antibodies were added for staining, and cells were distributed into wells of a 96-well round-bottom tissue culture plate. After incubation at 4°C for 45 minutes, 150 µL of FACS buffer was added to each well to wash the cells. Following centrifugation at 300 × g for 3 minutes, the supernatant was removed. After washing and resuspension in FACS buffer, cells were analyzed by flow cytometry.

### Enzyme-Linked Immunosorbent Assay

IL-15 concentration in culture supernatants was measured using ELISA MAX^{™} Deluxe Set Human IL-15 (Biolegend; 435104) according to the manufacturer's instructions.

### Experimental Results:

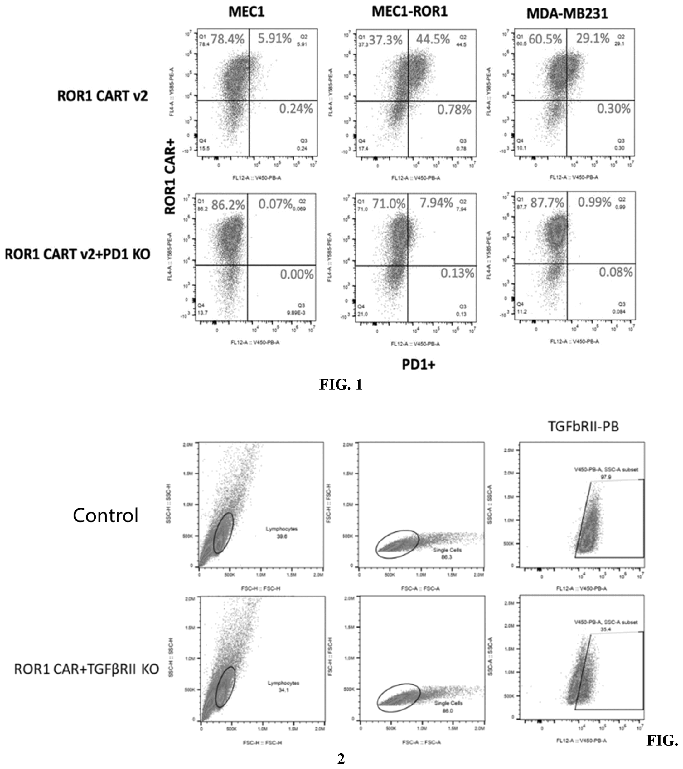

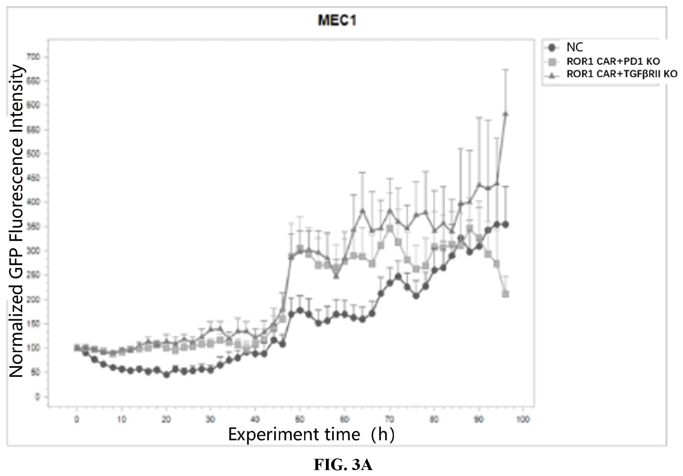

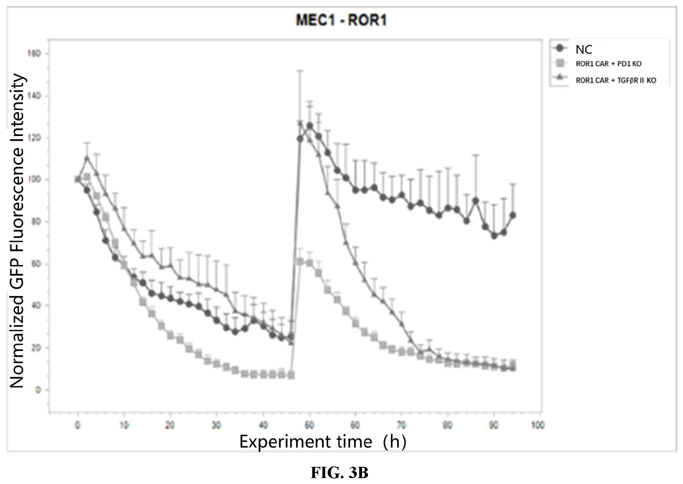

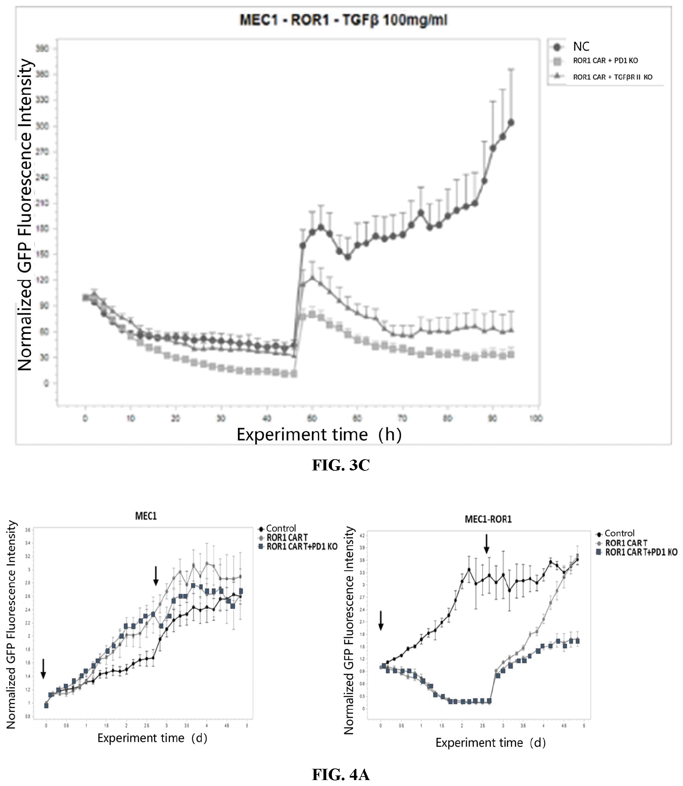

Expression of the relevant constructs is shown in Figure 1: over 90% of PD1 protein was successfully knocked out. Secretion of sIL15 and membrane expression of IL-15 are shown in Figure 3B, demonstrating normal secretion and expression.

### Example 2 Membrane Protein Intracellular Retention Technology Reduces PD1 Expression in CAR-iNKT Cells

This example investigated the efficacy of membrane protein intracellular retention technology in knocking out the PD1 gene in CAR-iNKT cells.

### 2.1 Tumor Cell Lines

The tumor cell line MEC1 (B-cell chronic lymphocytic leukemia) was engineered to generate a stable cell line expressing the ROR1 antigen. The MDA-MB-231 cell line inherently expresses the ROR1 antigen. These three tumor cell lines were transfected to express GFP and used in subsequent experiments.

### 2.2 Co-culture of CAR-T Cells with Tumor Cells

PBMCs transduced with ROR1 CAR v2 or ROR1 CAR v2-PD1 KO constructs and tumor cells were accurately counted, resuspended at a density of 1×10⁴ cells per well in 100 µL of cell culture medium at a 1:1 ratio, and seeded into round-bottom 96-well plates. After 72 hours of culture, cells were centrifuged and resuspended in FACS buffer, stained with PE-labeled ROR1 protein (Acro Biosystems; RO1-HP2E3) and BV421-labeled PD1 antibody (Biolegend; 367422), and analyzed by flow cytometry.

### Experimental Results:

As shown in Figure 1, PBMCs transduced with the ROR1 CARv2 construct induced PD1 expression upon co-culture with tumor cells (MEC1: 5.91%; MEC1-ROR1: 44.5%; MDA-MB-231: 29.1%).

PBMCs transduced with the ROR1 CARv2-PD1KO construct exhibited significantly reduced PD1 expression compared to non-knockout CAR-iNKT cells after co-culture with tumor cells (MEC1: 0.07%; MEC1-ROR1: 7.94%; MDA-MB-231: 0.99%).

These results demonstrate that the method provided in this application can generate stable iNKT cells lacking or exhibiting inhibited expression of negative immune regulator genes.

### Example 3 Membrane Protein Intracellular Retention Technology Reduces TGF-βR Expression in CAR-iNKT Cells

This example investigated the efficacy of membrane protein intracellular retention technology in knocking out the TGF-βR gene in CAR-iNKT cells.

### 3.1 Tumor Cell Lines

The tumor cell line MEC1 (B-cell chronic lymphocytic leukemia) was engineered to generate a stable cell line expressing the ROR1 antigen. The tumor cell lines were transfected to express GFP and used in subsequent experiments.

### 3.2 Validation of TGFβRII Knockout in CAR-iNKT Cells

After 72 hours of culture, cells were centrifuged and resuspended in FACS buffer, stained with PE-labeled ROR1 protein (Acro Biosystems; RO1-HP2E3) and BV421-labeled TGFβRII antibody (Biolegend; 399710), and analyzed by flow cytometry.

### 3.3 Re-challenge Assay of CAR-iNKT Cells with Tumor Cells (Incucyte Instrument-Based Assay)

PBMCs transduced with ROR1 CAR v2, ROR1 CAR v2-PD1 KO, or ROR1 CAR v2-TGFβRII KO constructs and MEC1 or MEC1-ROR1 tumor cells were accurately counted, resuspended at a density of 0.5×10⁴ cells per well in 100 µL of cell culture medium at a 1:1 ratio, seeded into flat-bottom 96-well plates, and placed into an Incucyte instrument to assess cytotoxicity (percentage of dead GFP-positive tumor cells) according to the manufacturer's instructions. In the TGFβ group, 100 ng/mL TGFβ was added during co-culture. After 48 hours of culture, 0.5×10⁴ tumor cells were re-added to the respective wells, and cytotoxicity was continuously monitored.

### Experimental Results:

As shown in Figure 2, the knockout efficiency of TGFβRII was approximately 60%.

Compared to PBMCs transduced with ROR1 CARv2-TGFβRIIKO, PBMCs transduced with ROR1 CARv2-PD1KO exhibited significantly superior cytotoxicity against MEC1-ROR1 tumor cells after co-culture (Figure 3B). Moreover, in groups treated with TGFβ to induce inhibition, PD1-knockout cells demonstrated excellent resistance to TGFβ-mediated inhibition, with significantly higher cytotoxicity than the TGFβR-knockout group.

These results indicate that PD1-knockout-enhanced CAR T cells described in this application exhibit superior efficacy compared to other approaches such as TGFβR knockout.

### Example 4 PD1-Knockout CAR-iNKT Cells Exhibit Enhanced Functionality

This example investigated the impact of PD1 gene knockout via membrane protein intracellular retention technology on the cytotoxicity and viability of CAR-iNKT cells.

### 4.1 Tumor Cell Lines

The tumor cell line MEC1 (B-cell chronic lymphocytic leukemia) was engineered to generate a stable cell line expressing the ROR1 antigen. The MDA-MB-231 (breast cancer) cell line inherently expresses the ROR1 antigen. These three tumor cell lines were transfected to express GFP and used in subsequent experiments.

### 4.2 Re-challenge Assay of CAR-iNKT Cells with Tumor Cells (Incucyte Instrument-Based Assay)

PBMCs transduced with ROR1 CAR v2 or ROR1 CAR v2-PD1 KO constructs and MEC1 or MEC1-ROR1 tumor cells were accurately counted, resuspended at a density of 0.25×10⁴ cells per well in 100 µL of cell culture medium at a 1:3 ratio, seeded into flat-bottom 96-well plates, and placed into an Incucyte instrument to assess cytotoxicity (percentage of dead GFP-positive tumor cells) according to the manufacturer's instructions. After 48 hours of culture, 0.75×10⁴ tumor cells were re-added to the respective wells, and cytotoxicity was continuously monitored.

### 4.3 Re-challenge Assay of CAR-iNKT Cells with Tumor Cells (Flow Cytometry-Based Assay)

PBMCs transduced with ROR1 CAR v2 or ROR1 CAR v2-PD1 KO constructs and MEC1, MEC1-ROR1, or MDA-MB-231 tumor cells were accurately counted, resuspended at a density of 0.25×10⁴ cells per well in 100 µL of cell culture medium at a 1:3 ratio, and seeded into three flat-bottom 96-well plates. Every three days, 0.75×10⁴ tumor cells were added to the remaining plates for continued culture. After 3, 7, and 11 days of culture, cells from one 96-well plate were centrifuged and resuspended in FACS buffer, stained with PE-labeled ROR1 protein (Acro Biosystems; RO1-HP2E3) and BV421-labeled PD1 antibody (Biolegend; 367422), and analyzed by flow cytometry.

### Experimental Results:

As shown in Figure 4A, compared to non-knockout ROR1-CAR-iNKT cells, PD1-knockout CAR-iNKT cells maintained robust inhibitory and cytotoxic functions even after secondary stimulation with tumor cells (D0, D3).

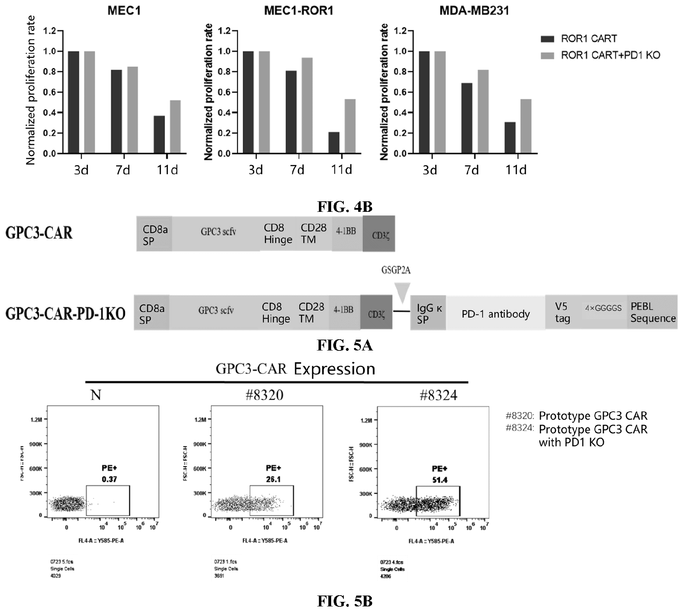

As shown in Figure 4B, compared to non-knockout CAR T cells, PD1-knockout CAR-iNKT cells exhibited significantly improved cell viability during in vitro co-culture.

These results demonstrate that the iNKT cells lacking or exhibiting inhibited expression of negative immune regulator genes, as provided in this application, display enhanced tumor-inhibitory activity and cytotoxic function, along with improved cell viability, following secondary stimulation with tumor cells, compared to unmodified iNKT cells. The modified iNKT cells simultaneously exhibited enhanced cytotoxic activity and prolonged survival-a synergistic effect that was unexpected and highlights the significant therapeutic potential of the iNKT cells described in this application for disease treatment.

### Example 5 Construction and Efficacy Evaluation of GPC3-CAR PD-1KO

### 5.1 Construction and Expression Analysis of GPC3-CAR

All relevant gene constructs were synthesized by Genewiz. GPC3 CAR-PD1KO was constructed by inserting the gene encoding GPC3 CAR--GSGP2A-PD1 antibody-PEBL into the pALD expression plasmid via SaII and SpeI cloning sites. To retain the PD1 protein intracellularly, an IgGk signal peptide (IgGKSP) was added to the N-terminus of the PD1 antibody (SEQ ID NO:19), and a PEBL sequence (LSM: SEQ ID NO:8) was appended to its C-terminus.

Using a third-generation lentiviral packaging system, a mixture of packaging plasmids, target plasmids, and PEIpro was transiently transfected into 293T cells. After 48 hours of culture, the lentivirus-containing supernatant was collected, filtered through a 0.45 µm filter, aliquoted at 1 mL per tube, and stored at -80°C. For use, the aforementioned lentivirus was thawed and transduced into iNKT cells. After 72 hours of culture, cells were stained with PE-labeled GPC3 protein (Acro Biosystems; GP3-HP2E3) and analyzed by flow cytometry to assess CAR construct expression.

Figure 5A illustrates the schematic constructs of GPC3-CAR and GPC3-CAR PD1KO.

### 5.2 In Vivo Efficacy Evaluation of GPC3-CAR and GPC3-CAR PD-1KO

### (1) Tumor Cell Culture and Implantation

Human hepatocellular carcinoma Hep3B cells were cultured in MEM medium supplemented with 10% FBS in a 37°C incubator with 5% CO₂. Prior to continuous passaging beyond ten generations, approximately 1×10⁶ Hep3B cells were resuspended in 100 µL PBS, mixed with an equal volume of Matrigel (v/v 1:1), and subcutaneously injected into the right flank of NCG-hIL15 mice at a total volume of 200 µL. Mice were anesthetized with 3-4% isoflurane prior to implantation.

### (2) Dosing Regimen

The day of tumor cell inoculation was defined as Day 0. Three days after tumor cell inoculation, mice were grouped according to body weight, with six mice per group, and subjected to corresponding dosing treatments. Animals received a dose of 1.5E7 total cells per animal, adjusted to a volume of 200 µL per animal, administered via tail vein injection, for a total of three doses on Days 3, 10, and 17 post-inoculation. Animals were monitored daily for general condition, and tumor diameters and body weights were measured twice weekly. At the end of the experiment, tumors from surviving animals were excised and weighed.

Experimental results: As shown in Figure 5B, the expression efficiency of the constructed GPC3-CAR PD1KO on iNKT cells was 50.1%, which is twice that of GPC3-CAR on iNKT cells (26.1%). GPC3-CAR PD1KO significantly enhanced the expression efficiency of GPC3-CAR on iNKT cells.

In vivo experimental results:

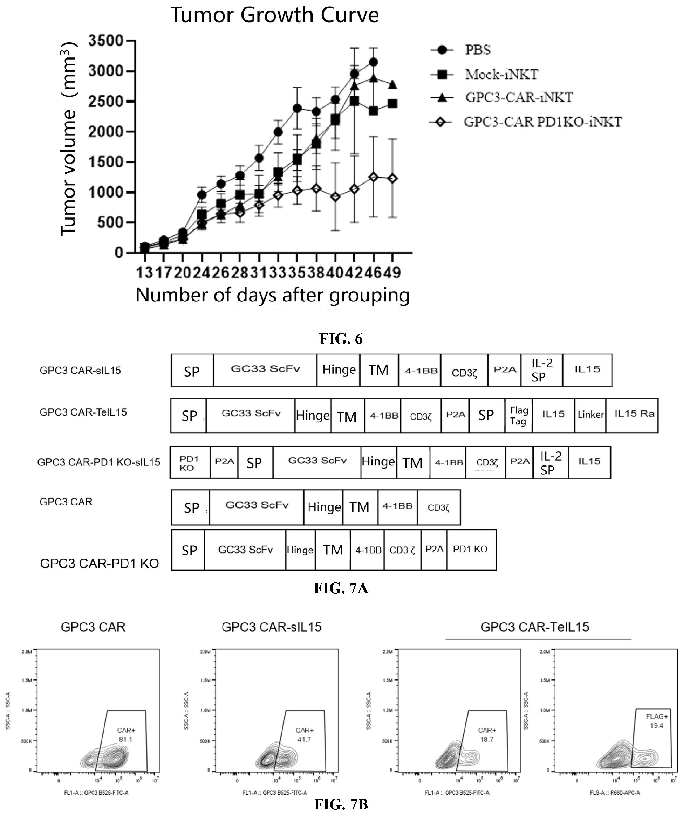

As shown in Figure 6, on Day 26, the mean tumor volumes for the PBS group, Mock-iNKT group, GPC3-CAR group, and GPC3-CAR PD1 KO group were 1141.9±124.51 mm³, 814.7±164.3 mm³, 635.6±79.8 mm³, and 645.8±146.3 mm³, respectively. Tumor volumes in the GPC3-CAR and GPC3-CAR PD1 KO groups were reduced compared to those in the PBS and Mock-iNKT groups, but no significant difference was observed between the two CAR-treated groups. By Day 42, the mean tumor volumes for the PBS group, Mock-iNKT group, GPC3-CAR group, and GPC3-CAR PD1 KO group were 2956.83±75.64 mm³, 2512±871.21 mm³, 2763.49±329.59 mm³, and 1056.6±553.9 mm³, respectively. On Day 42, the mean tumor volume in the GPC3-CAR PD1 KO group showed a statistically significant reduction (p<0.05) compared to the PBS, Mock-iNKT, and GPC3-CAR groups. These results indicate that, compared with GPC3-CAR, GPC3-CAR PD1 KO demonstrates a clear advantage in long-term antitumor efficacy.

The above results demonstrate that, compared with GPC3-CAR, GPC3-CAR PD1 KO not only enhances expression on iNKT cells but also significantly improves tumor inhibition.

### Example 6 Preparation of Tri-functional CAR Construct Combining PD1 Knockout and IL-15 Enhancement

The preparation procedure followed the steps below:

### 6.1 Construction of Tri-functional CAR Structure Combining PD1 Knockout and Interleukin-15 Enhancement

First, DNA sequences encoding different CAR structures were synthesized by a biotechnology company. In this invention, taking GPC3 CAR as an example, the design incorporating interleukin-15 to enhance CAR function involved either adding a secreted form of interleukin-15 after P2A or adding a membrane-tethered interleukin-15, as illustrated in Figure 7A. Based on the above structure, protein intracellular retention technology was simultaneously integrated, achieving both PD1 knockout and secretion of IL-15, resulting in a tri-functional GPC3-CAR architecture. Additionally, a standard second-generation GPC3 CAR was designed as an experimental control group. After obtaining synthetic plasmids containing the various CAR constructs, the designed structural fragments were inserted into the pALD plasmid using double restriction enzyme digestion for lentiviral packaging.

### 6.2 Expression of the Tri-functional CAR Structure Combining PD1 Knockout and IL-15 Enhancement

Using a third-generation lentiviral packaging system, a mixture of packaging plasmids, target plasmids, and Lipofectamine 3000 was transiently transfected into 293T cells. After 48 hours of culture, the lentivirus-containing supernatant was collected, filtered through a 0.45 µm filter, aliquoted into 1 mL tubes, and stored at -80°C. For use, the lentivirus carrying the aforementioned constructs was thawed and transduced into Jurkat cells or activated PBMCs. After 72 hours of culture, CAR expression was assessed by flow cytometry following staining with PE- or FITC-labeled GPC3 protein (Acro Biosystems; GP3-HP2E3 and GP3-HF2H1). For the membrane-tethered IL-15 construct, anti-FLAG tag antibody staining was additionally performed to confirm expression of the membrane-tethered IL-15 downstream of P2A. Additionally, supernatants from 72-hour cell cultures were collected and concentrated 4-10-fold using protein concentrator tubes as needed, followed by enzyme-linked immunosorbent assay (ELISA) to quantify IL-15 secreted into the culture medium.

### Experimental results:

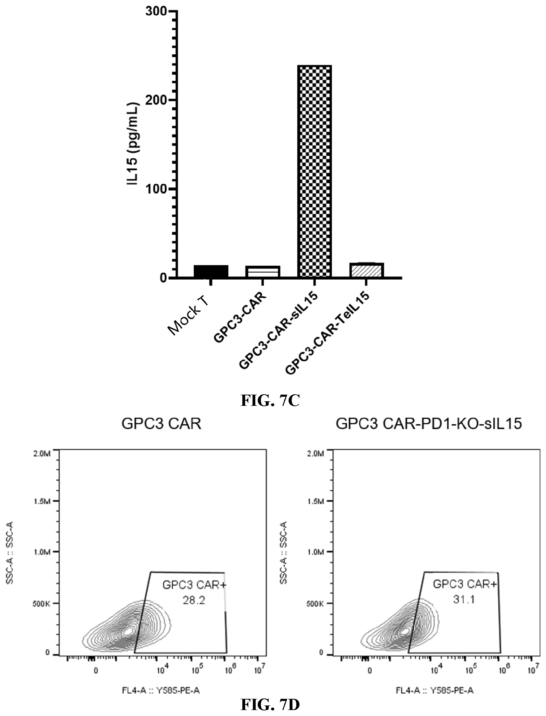

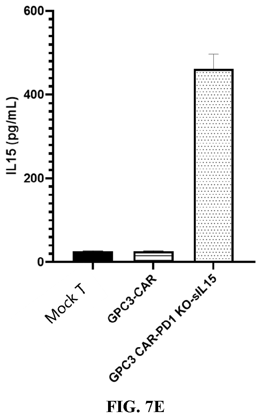

As shown in Figure 7B, GPC3-CAR, GPC3CAR-sIL15, and GPC3 CAR-TeIL15 were all normally expressed in PBMCs and bound antigen. In the supernatants collected 72 hours post-transduction, IL-15 secretion was detectable from the construct containing GPC3-CAR fused with secreted IL-15 (Figure 7C). Moreover, the tri-functional construct combining PD1 knockout and IL-15 enhancement successfully expressed the CAR structure in Jurkat cells (Figure 7D) and secreted IL-15 into the culture medium (Figure 7E).

These experimental results demonstrate that the iNKT cells provided in this application, which lack or have inhibited expression of negative immune regulator genes, can normally secrete cytokines after further engineering and, compared with unmodified iNKT cells, secrete greater amounts of cytokines within the same timeframe (Figure 7E). This effect was unexpected and indicates that the iNKT cells disclosed herein can be engineered to express various cytokines for disease treatment.

### Example 7 Secreted or Membrane-Tethered IL-15 Enhances CAR-T Cell Tumor-Killing Function

This example investigated the impact of incorporating either secreted IL-15 or membrane-tethered IL-15 on the tumor-killing function of CAR-iNKT cells.

### 7.1 Tumor Cell Lines

Hepatocellular carcinoma cell lines Hep3B and Huh7 were confirmed to endogenously express GPC3. Human breast cancer cell line MDA-MB-231 does not express GPC3. These three tumor cell lines were stably transfected to express GFP and used in subsequent experiments.

### 7.2 In vitro cytotoxicity assay of CAR-T cells (assessed by IncuCyte real-time imaging)

The aforementioned constructs (GPC3-CAR; GPC3-CAR-sIL15; and GPC3-CAR-TeIL15) were individually transduced into PBMCs. After 72 hours of culture, expression of each construct was confirmed by flow cytometry. Effector and target cells were precisely counted, and effector cell numbers were adjusted based on CAR expression rates to ensure equivalent CAR levels across all constructs. Cells were then resuspended in 200 µL of cell culture medium at an effector-to-target ratio of 1:3 and a cell density of 1 = 7×10³ cells, and seeded into flat-bottom 96-well plates. The 96-well plates were placed into an IncuCyte system for real-time imaging to monitor the 48-hour growth curves of GFP-labeled target cells. At the end of the cytotoxicity assay, the 96-well plates were removed, centrifuged, and supernatants were collected for ELISA to measure IFN-γ secretion.

### Experimental results:

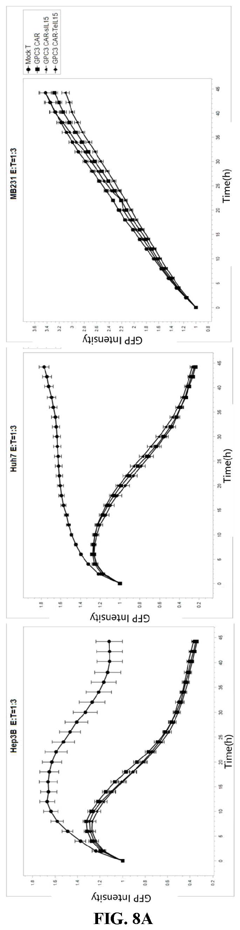

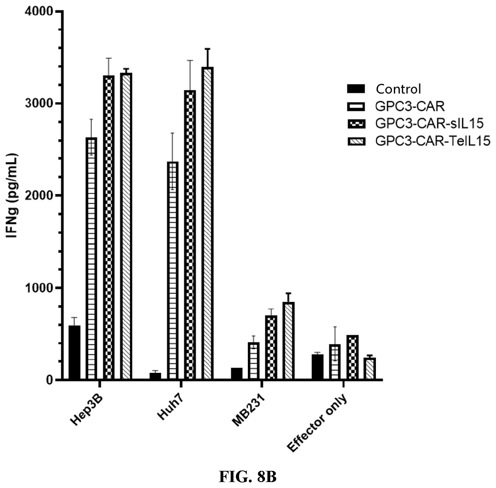

As shown in Figure 8A, against GPC3-expressing hepatocellular carcinoma target cells Hep3B and Huh7, both GPC3-CAR-T cells incorporating secreted IL-15 and those incorporating membrane-tethered IL-15 exhibited strong cytotoxic activity and tumor inhibition. No growth inhibition was observed against GPC3-negative MDA-MB-231 cells, demonstrating excellent specificity. In the IFN-γ secretion assay (Figure 8B), constructs incorporating either secreted or membrane-tethered IL-15 showed elevated IL-15 secretion levels, reflecting enhanced tumor-killing capacity.

These experimental results demonstrate that unmodified iNKT cells can be engineered via different configurations to express cytokines and exhibit potent tumor-killing activity.

### Example 8 Tri-functional Construct Combining PD1 Knockout and IL-15 Enhancement Reduces PD1 Expression in CAR-T Cells

This example examined the efficacy of PD1 knockout achieved by the tri-functional construct combining PD1 knockout and IL-15 enhancement during tumor killing.

### 8.1 Tumor Cell Lines

Hepatocellular carcinoma cell lines Hep3B and Huh7 were confirmed to endogenously express GPC3. Human breast cancer cell line MDA-MB-231 does not express GPC3. These three tumor cell lines were stably transfected to express GFP and used in subsequent experiments.

### 8.2 Co-culture of CAR-iNKT cells with tumor cells and flow cytometric analysis of PD1 expression

The aforementioned constructs (GPC3-CAR; GPC3-CAR-PD1 KO-sIL15) were individually transduced into Jurkat cells. After 72 hours of culture, CAR expression was assessed by flow cytometry, and cell densities were adjusted to achieve equivalent CAR expression rates. Effector and target cells were precisely counted and then resuspended in 200 µL of cell culture medium at an effector-to-target ratio of 1:1 and a cell density of 1 = 5×10⁴ cells, and seeded into round-bottom 96-well plates. After 72 hours of co-culture, cells were washed with PBS and stained with PE-labeled GPC3 protein (Acro Biosystems; GP3-HP2E3) and BV421-labeled PD1 antibody (BioLegend; 367422), followed by flow cytometric analysis.

### Experimental results:

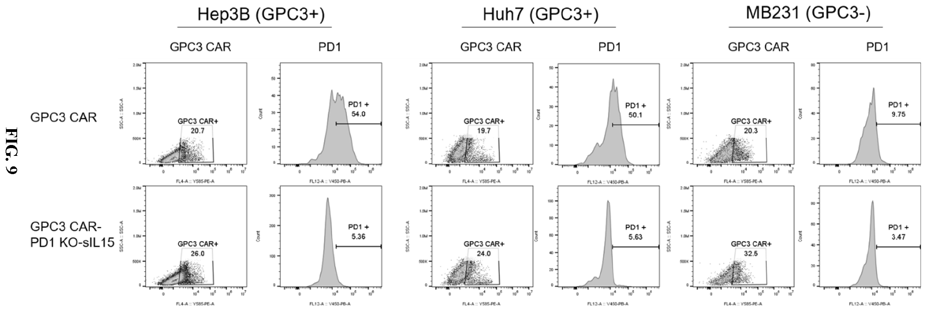

As shown in Figure 9, after co-culture with tumor cells, the GPC3-CAR group induced high levels of PD1 expression, particularly in CAR-T cells co-cultured with specific target cells, which exhibited approximately 50% PD1 expression. In contrast, our tri-functional CAR-T architecture, employing PD1 membrane protein retention technology, significantly reduced PD1 expression on the cell surface, demonstrating effective knockout. This renders CAR-iNKT cells less prone to exhaustion and confers more durable tumor-killing activity.

These experimental results demonstrate that, compared with unmodified iNKT cells, the iNKT cells provided in this application-lacking or exhibiting inhibited expression of negative immune regulator genes-exhibit more sustained tumor-killing efficacy.

## Claims

1. A modified iNKT cell, which, compared to an unmodified iNKT cell, lacks or inhibits expression of one or more negative immune regulator genes selected from the group consisting of: PD-1, Tim-3, CTLA-4, CD74, and TGF-βR, wherein said lack or inhibition of expression of the negative immune regulator genes are achieved by membrane protein intracellular retention/degradation technology.

2. The iNKT cell of claim 1, wherein said membrane protein intracellular retention technology involves directly or indirectly linking a targeting sequence to the N-terminus or C-terminus of the negative immune regulator gene.

3. The iNKT cell of any one of claims 1-2, wherein said membrane protein intracellular retention technology involves directly or indirectly linking a targeting sequence to the C-terminus of the negative immune regulator gene.

4. The iNKT cell of any one of claims 1-3, wherein said membrane protein intracellular retention technology involves directly or indirectly linking a targeting sequence to the N-terminus of the negative immune regulator gene.

5. The iNKT cell of any one of claims 2-4, wherein said indirect linkage uses (GGGGS)₄ as a linker.

6. The iNKT cell of any one of claims 1-5, wherein said negative immune regulator gene is PD-1.

7. The iNKT cell of claim 6, wherein the amino acid sequence of said PD-1 is as set forth in SEQ ID NO: 12 or SEQ ID NO: 19.

8. The iNKT cell of any one of claims 2-7, wherein said targeting sequence is selected from the group consisting of: endoplasmic reticulum retention sequence, Golgi retention sequence, E3 ubiquitin ligase binding sequence, proteasome targeting sequence, and lysosomal targeting sequence.

9. The iNKT cell of claim 8, wherein said targeting sequence is an endoplasmic reticulum retention sequence or a lysosomal targeting sequence.

10. The iNKT cell of any one of claims 8-9, wherein the amino acid sequence of said endoplasmic reticulum retention sequence comprises one or more amino acid sequences optionally selected from the group consisting of: KDEL (SEQ ID NO: 9), KKMP (SEQ ID NO: 10), and AEKDEL (SEQ ID NO: 11).

11. The iNKT cell of any one of claims 8-9, wherein the amino acid sequence of said lysosomal targeting sequence is as shown in SFHDDSDEDLLHI (SEQ ID NO: 8).

12. The iNKT cell of any one of claims 1-11, wherein said iNKT cell expresses a CAR.

13. The iNKT cell of claim 12, wherein said CAR comprises a targeting moiety that specifically binds to an antigen.

14. The iNKT cell of claim 13, wherein said antigen is selected from one or more antigens from the group consisting of: GPC3, CLDN18.2, ROR1, and AXL.

15. The iNKT cell of any one of claims 12-14, wherein said antigen is GPC3 or ROR1.

16. The iNKT cell of any one of claims 12-14, wherein said targeting moiety is an scFv.

17. The iNKT cell of any one of claims 12-14, wherein said targeting moiety is a VHH.

18. The iNKT cell of any one of claims 12-17, wherein said CAR comprises a signal peptide.

19. The iNKT cell of claim 18, wherein said signal peptide is a signal peptide derived from CD8 protein.

20. The iNKT cell of claim 18, wherein said signal peptide is a signal peptide derived from IL-2.

21. The iNKT cell of any one of claims 12-20, wherein said CAR comprises a hinge region, said hinge region comprising a hinge region derived from one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

22. The iNKT cell of claim 21, wherein said hinge region is a hinge region derived from CD28 or CD8.

23. The iNKT cell of any one of claims 12-22, wherein said CAR comprises a transmembrane domain, said transmembrane domain comprising a transmembrane domain derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

24. The iNKT cell of claim 23, wherein said transmembrane domain is a transmembrane domain derived from CD28.

25. The iNKT cell of any one of claims 12-24, wherein said CAR comprises a co-stimulatory domain, said co-stimulatory domain comprising a co-stimulatory domain derived from one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, ligand of CD83, CD40, and MyD88.

26. The iNKT cell of claim 23, wherein said co-stimulatory domain is a co-stimulatory domain derived from 4-1BB.

27. The iNKT cell of any one of claims 12-25, wherein said CAR comprises an intracellular signaling domain, said intracellular signaling domain comprising an intracellular signaling domain derived from one or more proteins selected from the group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP12, and a domain containing at least one ITAM.

28. The iNKT cell of claim 27, wherein said intracellular signaling domain is an intracellular signaling domain derived from CD3ζ.

29. The iNKT cell of any one of claims 1-28, wherein said iNKT cell is a CAR-iNKT cell, and the CAR consists of, linked sequentially: CD8 signal peptide, GPC3 antibody, CD8 hinge region, CD28 transmembrane domain, 4-1BB co-stimulatory domain, CD3ζ intracellular signaling domain, GSG linker sequence, P2A self-cleaving peptide, IgGκ signal peptide, PD-1 antibody, V5 tag, (GGGGS)₄, and targeting sequence.

30. The iNKT cell of any one of claims 1-28, wherein said iNKT cell is a CAR-iNKT cell, and the CAR consists of, linked sequentially: CD8 signal peptide, ROR1 antibody, CD28 hinge region, CD28 transmembrane domain, 4-1BB co-stimulatory domain, CD3ζ intracellular signaling domain, GSG linker sequence, P2A self-cleaving peptide, IgGκ signal peptide, PD-1 antibody, V5 tag, (GGGGS)₄, and targeting sequence.

31. The CAR-iNKT cell of any one of claims 29-30, wherein the amino acid sequence of said PD-1 antibody is as set forth in SEQ ID NO: 19, and the amino acid sequence of said targeting sequence is as set forth in SEQ ID NO: 8.

32. The iNKT cell of any one of claims 12-31, wherein said CAR comprises a cytokine domain.

33. The iNKT cell of claim 32, wherein said cytokine domain:
(1) comprises one or more cytokines and/or functional fragments thereof;
(2) comprises one or more cytokine receptors and/or functional fragments thereof; and
(3) comprises a combination of (1) and (2).

34. The iNKT cell of any one of claims 32-33, wherein said cytokine domain is expressed on the iNKT cell membrane.

35. The iNKT cell of any one of claims 32-34, wherein said cytokine domain is secreted from the iNKT cell.

36. The iNKT cell of any one of claims 32-35, wherein said cytokine is selected from one or more factors from the group consisting of: IL-15, IL-10, IL-18, and IL-21.

37. The iNKT cell of any one of claims 32-36, wherein said cytokine is IL-15.

38. The iNKT cell of any one of claims 32-36, wherein said cytokine receptor is IL-15R or its alpha subunit (IL-15Rα).

39. A nucleic acid molecule encoding a sequence comprising a nucleic acid sequence encoding a negative immune regulator gene and a nucleic acid sequence encoding a targeting sequence, wherein said negative immune regulator gene is selected from one or more genes from the group consisting of: PD-1, Tim-3, CTLA-4, CD74, and TGF-βR.

40. The nucleic acid molecule of claim 39, wherein said negative immune regulator gene is PD-1.

41. The nucleic acid molecule of any one of claims 39-40, wherein the amino acid sequence of said PD-1 is as set forth in SEQ ID NO: 12 or SEQ ID NO: 19.

42. The nucleic acid molecule of any one of claims 39-41, wherein said targeting sequence is selected from the group consisting of: endoplasmic reticulum retention sequence, Golgi retention sequence, E3 ubiquitin ligase binding sequence, proteasome targeting sequence, and lysosomal targeting sequence.

43. The nucleic acid molecule of any one of claims 39-42, wherein said targeting sequence is an endoplasmic reticulum retention sequence or a lysosomal targeting sequence.

44. The nucleic acid molecule of any one of claims 42-43, wherein the amino acid sequence of said endoplasmic reticulum retention sequence comprises one or more amino acid sequences optionally selected from the group consisting of: KDEL (SEQ ID NO: 9), KKMP (SEQ ID NO: 10), and AEKDEL (SEQ ID NO: 11).

45. The nucleic acid molecule of any one of claims 42-43, wherein the amino acid sequence of said lysosomal targeting sequence is as shown in SFHDDSDEDLLHI (SEQ ID NO: 8).

46. The nucleic acid molecule of any one of claims 39-45, which comprises a nucleic acid sequence encoding an antigen-targeting moiety.

47. The nucleic acid molecule of claim 46, wherein said antigen is selected from one or more antigens from the group consisting of: GPC3, CLDN18.2, ROR1, and AXL.

48. The nucleic acid molecule of any one of claims 46-47, wherein said antigen is GPC3 or ROR1.

49. The nucleic acid molecule of any one of claims 39-48, which comprises a nucleic acid sequence encoding a promoter.

50. The nucleic acid molecule of claim 49, wherein said promoter is selected from the group consisting of: EF1α, CMV, MSCV, SSFV, and UbC.

51. The nucleic acid molecule of any one of claims 39-50, which comprises a nucleic acid sequence encoding a signal peptide.

52. The nucleic acid molecule of claim 51, wherein said signal peptide is a signal peptide derived from CD8 protein.

53. The nucleic acid molecule of claim 51, wherein said signal peptide is a signal peptide derived from IL-2.

54. The nucleic acid molecule of any one of claims 39-53, which comprises a nucleic acid sequence encoding a hinge region, said hinge region comprising a hinge region derived from one or more proteins selected from the group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

55. The nucleic acid molecule of claim 54, wherein said hinge region is a hinge region derived from CD28 or CD8.

56. The nucleic acid molecule of any one of claims 39-55, which comprises a nucleic acid sequence encoding a transmembrane domain, said transmembrane domain comprising a transmembrane domain derived from one or more proteins selected from the group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

57. The nucleic acid molecule of claim 56, wherein said transmembrane domain is a transmembrane domain derived from CD28.

58. The nucleic acid molecule of any one of claims 39-57, which comprises a nucleic acid sequence encoding a co-stimulatory domain, said co-stimulatory domain comprising a co-stimulatory domain derived from one or more proteins selected from the group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, ligand of CD83, CD40, and MyD88.

59. The nucleic acid molecule of claim 58, wherein said co-stimulatory domain is an intracellular co-stimulatory domain derived from 4-1BB.

60. The nucleic acid molecule of any one of claims 39-59, which comprises a nucleic acid sequence encoding an intracellular signaling domain, said intracellular signaling domain comprising an intracellular signaling domain derived from one or more proteins selected from the group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpesvirus (HSKV), DAP10, DAP12, and a domain containing at least one ITAM.

61. The nucleic acid molecule of claim 60, wherein said intracellular signaling domain is an intracellular signaling domain derived from CD3ζ.

62. The nucleic acid molecule of any one of claims 39-61, wherein said targeting sequence is located upstream of the sequence of said negative immune regulator gene.

63. The nucleic acid molecule of any one of claims 39-61, wherein said targeting sequence is located downstream of the sequence of said negative immune regulator gene.

64. The nucleic acid molecule of any one of claims 39-63, which comprises a sequence encoding a cleavage peptide.

65. The nucleic acid molecule of claim 64, wherein said cleavage peptide is selected from one or more cleavage peptides from the group consisting of: P2A, T2A, F2A, E2A, BmCPV2A, IRES, and BmIFV2A.

66. The nucleic acid molecule of any one of claims 64-65, wherein said cleavage peptide is P2A.

67. The nucleic acid molecule of any one of claims 64-66, wherein said cleavage peptide P2A is linked to the linker sequence GSG.

68. The nucleic acid molecule of any one of claims 39-67, which can encode, sequentially: CD8 signal peptide, GPC3 antibody, CD8 hinge region, CD28 transmembrane domain, 4-1BB co-stimulatory domain, CD3ζ intracellular signaling domain, GSG linker sequence, P2A self-cleaving peptide, IgGκ signal peptide, PD-1 antibody, V5 tag, (GGGGS)₄, and targeting sequence.

69. The nucleic acid molecule of any one of claims 39-67, which can encode, sequentially: CD8 signal peptide, ROR1 antibody, CD28 hinge region, CD28 transmembrane domain, 4-1BB co-stimulatory domain, CD3ζ intracellular signaling domain, GSG linker sequence, P2A self-cleaving peptide, IgGκ signal peptide, PD-1 antibody, V5 tag, (GGGGS)₄, and targeting sequence.

70. The nucleic acid molecule of any one of claims 68-69, wherein the amino acid sequence of said PD-1 antibody is as set forth in SEQ ID NO: 19, and the amino acid sequence of said targeting sequence is as set forth in SEQ ID NO: 8.

71. The nucleic acid molecule of any one of claims 39-70, which comprises a nucleic acid sequence encoding a cytokine domain.

72. The nucleic acid molecule of claim 71, wherein said nucleic acid sequence encoding a cytokine domain may be:
(1) a sequence capable of expressing one or more cytokines and/or functional fragments thereof;
(2) a sequence capable of expressing one or more cytokine receptors and/or functional fragments thereof; and
(3) a sequence capable of expressing a combination of (1) and (2).

73. The nucleic acid molecule of any one of claims 71-72, wherein said cytokine is selected from one or more factors from the group consisting of: IL-15, IL-10, IL-18, and IL-21.

74. The nucleic acid molecule of any one of claims 71-73, wherein said cytokine is IL-15.

75. The nucleic acid molecule of any one of claims 71-73, wherein said cytokine receptor is IL-15R or its alpha subunit (IL-15Rα).

76. A plasmid comprising the nucleic acid molecule of any one of claims 39-75.

77. The plasmid of claim 76, which includes viral plasmids and bacterial plasmids.

78. The plasmid of claim 76, which includes DNA plasmids and RNA plasmids.

79. The plasmid of claim 76, which includes linear plasmids and circular plasmids.

80. A pharmaceutical composition comprising the iNKT cell of any one of claims 1-38, the nucleic acid molecule of any one of claims 39-75, the plasmid of any one of claims 76-79, and at least one pharmaceutically acceptable carrier.

81. Use of the iNKT cell of any one of claims 1-38, the nucleic acid molecule of any one of claims 39-75, the plasmid of any one of claims 76-79, and the pharmaceutical composition of claim 80 in the manufacture of a medicament for the prevention, alleviation, and/or treatment of a disease and/or disorder.
